# FASCICULE DE BREVET EUROPEEN

(11) **EP 3 151 787 B1**
(45) Date de publication et mention de la délivrance du brevet: **10.03.2021**
(21) Numéro de dépôt: 15733811.2
(22) Date de dépôt: 05.06.2015
(51) Int. Cl.: A61F 2/42, A61F 2/46

(54) **IMPLANT CHIRURGICAL, OUTIL DE MISE EN PLACE, KIT CHIRURGICAL ET PROCEDE DE FABRICATION AFFERENTS**
CHIRURGISCHES IMPLANTAT UND ZUGEHÖRIGES INSTALLATIONSWERKZEUG, CHIRURGISCHES KIT UND VERFAHREN ZUR HERSTELLUNG
SURGICAL IMPLANT, AND ASSOCIATED INSTALLATION TOOL, SURGICAL KIT AND METHOD OF PRODUCTION

(30) Priorité: 06.06.2014 FR 1455169
(43) Date de publication de la demande: 12.04.2017
(73) Titulaire: In2Bones, 69130 Ecully (FR)
(72) Inventeur: VILADOT PERICE, Ramon, E-08022 Barcelone (ES); VILADOT VOEGELI, Antonio, E-08022 Barcelone (ES)
(74) Mandataire: Weber, Jean-François
(86) Numéro de dépôt international: PCT/FR2015/051499
(87) Numéro de publication internationale: WO 2015/185876

(56) Documents cités:
- EP-A1- 1 728 491
- WO-A1-97/25940
- WO-A1-97/30660
- US-A- 6 136 032

## Description

### DOMAINE TECHNIQUE

La présente invention concerne le domaine des prothèses chirurgicales, et en particulier des implants chirurgicaux destinés à effectuer une correction du positionnement ou de l'orientation mutuel(le) des os d'un patient, notamment dans le cadre du traitement d'une pathologie du pied plat, et à être insérés à cet effet entre les corps osseux concernés.

L'invention concerne plus précisément un implant chirurgical conçu pour être inséré entre au moins deux corps osseux d'un patient, ledit implant chirurgical comprenant :
- un corps principal s'étendant le long d'un axe longitudinal entre une extrémité proximale et une extrémité distale dudit corps principal,
- une pluralité d'ailettes, chaque ailette étant pourvue d'un bord de jonction reliant ladite ailette audit corps principal, de sorte que lesdites ailettes font saillie dudit corps principal à partir de leur bord de jonction respectif, lequel s'étend sensiblement dans un plan de jonction dont la normale est formée par ledit axe longitudinal.

L'invention concerne également un outil de mise en place d'un implant chirurgical.

L'invention concerne en outre un kit chirurgical incluant :
- un implant chirurgical,
- un outil de mise en place de l'implant chirurgical.

L'invention concerne enfin un procédé de fabrication d'un implant chirurgical.

### TECHNIQUE ANTERIEURE

On connait des implants chirurgicaux, lesquels sont prévus pour être fixés dans la cavité du sinus tarsien. De tels implants, en soutenant et en repositionnant les os concernés à la manière d'une cale, permettent de reformer la voûte plantaire naturelle d'un patient souffrant d'une pathologie de pied plat. De manière générale, ces implants connus comportent en particulier une couronne externe sensiblement conique destinée à entrer en contact avec la cavité du sinus du tarse. Cette couronne externe est, le plus souvent, pourvue sur sa face externe d'éléments anti-retours saillants, du genre d'ailettes anti-recul, conçus pour s'opposer à l'expulsion de l'implant lorsqu'il est en place dans ladite cavité. De tels implants donnent généralement satisfaction et sont susceptibles, le plus souvent, de permettre l'amélioration de la condition, ou même la guérison, des patients souffrant de la pathologie du pied plat.

Toutefois, certains de ces implants chirurgicaux, lorsqu'ils sont implantés dans la cavité du sinus tarsien, peuvent être susceptibles de causer certaines douleurs pour le patient, laquelle peut s'expliquer par la pression qu'ils sont amenés à exercer sur les corps osseux du pied à traiter. Également, les ailettes de certains de ces implants connus peuvent parfois causer des lésions de la surface des corps osseux avec lesquels elles sont en contact, par exemple dans le cas où la couronne et les ailettes sont réalisées dans un matériau métallique. Néanmoins, l'absence d'éléments saillants nuirait à la propension de l'implant à se maintenir de façon fiable dans la cavité osseuse, ce qui non seulement serait susceptible de rendre le traitement inefficace, mais pourrait également représenter un risque pour la santé du patient.

La couronne peut également être réalisée en polyéthylène, ce qui peut rendre difficile son repérage et sa détection dans le corps du patient à l'aide d'une radiographie. Il peut ainsi être malaisé de contrôler le positionnement de l'implant dans la cavité, et l'évolution du traitement. La couronne, si elle est au contraire réalisée en matière métallique, est susceptible de bloquer le rayonnement X et d'entraîner, sur la radiographie, un masquage par l'implant la zone du corps à traiter.

Les implants chirurgicaux connus de ce type sont en outre généralement formés par un assemblage de plusieurs pièces, en particulier d'un assemblage de la couronne périphérique avec un cône d'extension central, qui nécessitent d'être réalisés soigneusement de façon indépendante afin d'être ensuite assemblés. De ce fait, il semble que la conception de ces implants pourrait être améliorée afin de réduire le nombre d'opérations de fabrication et d'assemblage, et par conséquent le prix de revient de chaque implant.

Le document EP-1 728 491 A1 décrit un dispositif chirurgical de traitement du pied plat comprenant au moins deux composants, à savoir une enveloppe externe et un organe d'expansion, apte à se déplacer au sein de l'enveloppe externe suivant une direction de compression, pour entraîner l'expansion radiale de l'enveloppe externe, ce dispositif comportant des moyens de blocage anti-retour, conformés pour autoriser le déplacement de l'organe d'expansion, selon la direction de compression, vers une position fonctionnelle au sein de l'enveloppe externe et s'opposer au déplacement en sens inverse de l'organe d'expansion une fois la position fonctionnelle atteinte, de manière à empêcher l'expulsion de l'organe d'expansion.

### EXPOSE DE L'INVENTION

Les objets assignés à la présente invention visent en conséquence à remédier aux différents inconvénients énumérés précédemment et à proposer un nouvel implant chirurgical, un nouvel outil de mise en place, un nouveau kit chirurgical, et un nouveau procédé de fabrication, permettant d'effectuer une correction du positionnement ou de l'orientation mutuel(le) des corps osseux efficace tout en limitant la douleur subie par le patient.

Un autre objet de l'invention vise à proposer un nouvel implant chirurgical de correction du positionnement ou de l'orientation mutuel(le) des os d'un patient, un nouvel outil de mise en place, un nouveau kit chirurgical, et un nouveau procédé de fabrication, particulièrement polyvalents, et en l'espèce adaptés à toutes les formes de corps osseux.

Un autre objet de l'invention vise à proposer un nouvel implant chirurgical de correction du positionnement ou de l'orientation mutuel(le) des os d'un patient, un nouvel outil de mise en place, un nouveau kit chirurgical, et un nouveau procédé de fabrication, dont la mise en œuvre et la mise en place sont particulièrement aisées.

Un autre objet de l'invention vise à proposer un nouvel implant chirurgical de correction du positionnement ou de l'orientation mutuel(le) des os d'un patient, un nouvel outil de mise en place, un nouveau kit chirurgical, et un nouveau procédé de fabrication, grâce auxquels il est facile de contrôler l'évolution du traitement du patient.

Un autre objet de l'invention vise à proposer un nouvel implant chirurgical de correction du positionnement ou de l'orientation mutuel(le) des os d'un patient, un nouvel outil de mise en place, un nouveau kit chirurgical, et un nouveau procédé de fabrication, dont la fabrication est facilitée et le coût est réduit.

Un autre objet de l'invention vise à proposer un nouvel implant chirurgical de correction du positionnement ou de l'orientation mutuel(le) des os d'un patient, un nouvel outil de mise en place, un nouveau kit chirurgical, et un nouveau procédé de fabrication, permettant de traiter efficacement des pathologies liées à un défaut d'orientation de corps osseux, en particulier la pathologie du pied plat.

Un autre objet de l'invention vise à proposer un nouvel implant chirurgical de correction du positionnement ou de l'orientation mutuel(le) des os d'un patient, un nouvel outil de mise en place, un nouveau kit chirurgical, et un nouveau procédé de fabrication, permettant une mise en place particulièrement fiable de l'implant dans le corps du patient.

Un autre objet de l'invention vise à proposer un nouvel implant chirurgical de correction du positionnement ou de l'orientation mutuel(le) des os d'un patient, un nouvel outil de mise en place, un nouveau kit chirurgical, et un nouveau procédé de fabrication, qui n'est pas susceptible de causer des lésions corporelles sur le patient, notamment sur les corps osseux du patient.

Les objets assignés à l'invention sont atteints à l'aide d'un implant chirurgical selon la revendication 1.

Les objets assignés à l'invention sont également atteints à l'aide d'un outil de mise en place d'un implant chirurgical selon l'invention, l'outil de mise en place comprenant :
- une tête conçue pour coopérer à rotation avec l'implant chirurgical autour de l'axe longitudinal de ce dernier, et
- une poignée de préhension par l'intermédiaire de laquelle un utilisateur peut saisir ledit outil de mise en place, ladite poignée comportant un témoin visuel de l'orientation de l'implant chirurgical autour de son axe longitudinal lorsque ledit outil de mise en place est en coopération avec ledit implant chirurgical.

Les objets assignés à l'invention sont atteints en outre à l'aide d'un kit chirurgical incluant :
- un implant chirurgical selon l'invention,
- un outil de mise en place de l'implant chirurgical.

Les objets assignés à l'invention sont en outre atteints à l'aide d'un procédé de fabrication d'un implant chirurgical selon l'invention, caractérisé en ce qu'il comprend :
- soit une étape de moulage d'une pièce finie monobloc destinée à former l'implant chirurgical,
- soit une étape d'usinage d'une pièce brute monobloc pour former une pièce finie monobloc destinée à former elle-même l'implant chirurgical.

Enfin, les objets assignés à l'invention sont mis en place à l'aide d'un procédé de mise en place d'un implant chirurgical avec un outil de mise en place selon l'invention, caractérisé en ce que le procédé comprend :
- une étape au cours de laquelle on solidarise l'implant chirurgical à l'outil de mise en place, en faisant interagir la tête de l'outil de mise en place avec l'ouverture traversante de l'implant chirurgical
- une étape au cours de laquelle on insère l'implant chirurgical entre les corps osseux du patient souhaités en tenant ledit implant chirurgical par l'intermédiaire de l'outil de mise en place
- une étape au cours de laquelle on règle avantageusement l'orientation de l'implant chirurgical autour de l'axe longitudinal afin d'ajuster sa forme générale à la forme de la cavité ménagée entre les corps osseux par rotation autour dudit axe longitudinal de l'outil de mise en place
- une étape au cours de laquelle on désolidarise l'implant chirurgical de l'outil de mise en place, en dévissant l'élément de vissage du filetage intérieur, et en séparant la tête de l'ouverture traversante.

### DESCRIPTIF SOMMAIRE DES DESSINS

D'autres particularités et avantages de l'invention apparaîtront et ressortiront plus en détails à la lecture de la description faite ci-après, en référence aux dessins annexés, donnés uniquement à titre d'exemple illustratif et non limitatif, dans lesquels :
- Les figures 1 et 2 illustrent, selon des vues en perspective, un implant chirurgical conforme à l'invention.
- Les figures 3 et 4 représentent, selon des vues de côté, l'implant chirurgical des figures 1 et 2.
- La figure 5 illustre, selon une vue de côté orientée de la même façon que celle de la figure 4, l'implant chirurgical des figures 1 à 4 en coupe longitudinale.
- La figure 6 illustre, selon une vue de côté orientée de la même façon que celle de la figure 3, l'implant chirurgical des figures 1 à 5 en coupe longitudinale.
- La figure 7 illustre, selon une vue en perspective, l'implant chirurgical des figures 1 à 6 inséré entre deux corps osseux du pied d'un patient.
- La figure 8 représente, selon une vue de côté, l'implant chirurgical des figures 1 à 7 associé à un outil de mise en place conforme à l'invention, pour former un kit chirurgical conforme à l'invention.
- La figure 9 représente, selon une vue de côté en coupe longitudinale, l'outil de mise en place de la figure 8.
- La figure 10 illustre, selon une vue en perspective coupée longitudinalement, un détail de réalisation de l'outil de mise en place des figures 8 et 9.
- La figure 11 représente, selon une vue en perspective orientée de la même manière que celle de la figure 10, le détail de réalisation de l'outil de mise en place de la figure 10.

### MEILLEURE MANIERE DE REALISER L'INVENTION

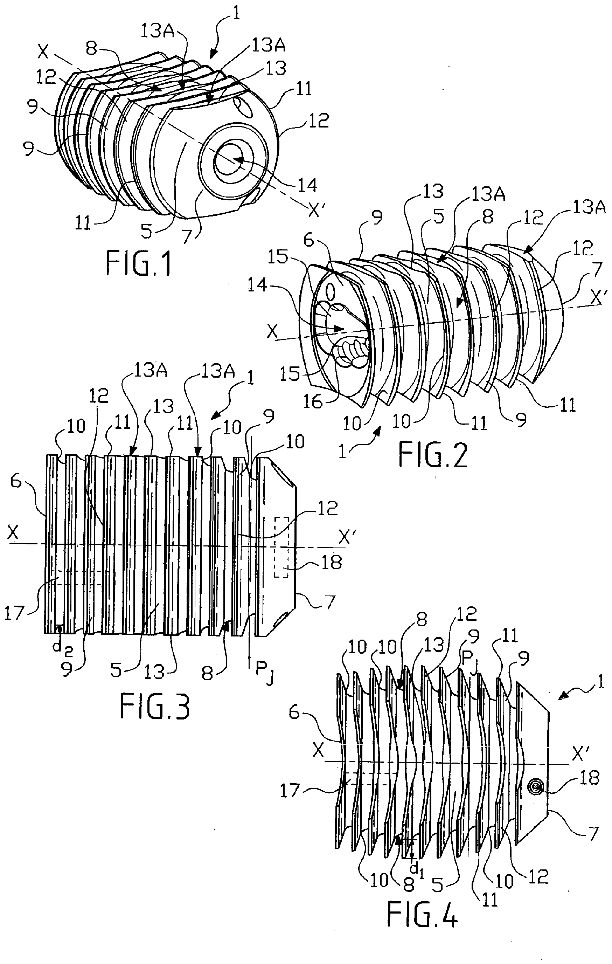

L'invention concerne en tant que tel un implant chirurgical 1 de correction du positionnement ou de l'orientation mutuel(le) des os d'un patient, dont un exemple de réalisation non limitatif et non exhaustif est illustré aux figures 1 à 7. Cet implant chirurgical 1 est conçu pour être introduit à l'intérieur du corps d'un patient, en particulier au cours d'une opération chirurgicale impliquant par exemple une anesthésie au moins locale dudit patient, et une incision à travers la peau du patient à proximité de la zone où l'implant chirurgical 1 doit être implanté, afin d'insérer ledit implant chirurgical 1 à travers l'incision jusqu'à ladite zone à l'intérieur du corps du patient.

L'implant chirurgical 1 de l'invention a pour fonction principale d'interagir avec des corps osseux 2, 3 du patient, en particulier pour effectuer une correction du positionnement, c'est-à-dire l'orientation relative ou absolue, desdits corps osseux 2, 3.

Au sens de l'invention, on entend par « *corps osseux* » 2, 3, des éléments du squelette du patient, tels que des os, du cartilage, ou une combinaison de ces derniers. Au sens de l'invention, les corps osseux 2, 3 sont des éléments qui contribuent à former une charpente rigide et articulée du corps du patient, tels que des os du bras, de la main, de la colonne vertébrale, du pied, du crâne etc. Le terme « *corps osseux* » désigne en particulier des os courts du patient, tels que des carpes ou des tarses, ou des os intermédiaires, tels que des métacarpiens, ou des métatarsiens.

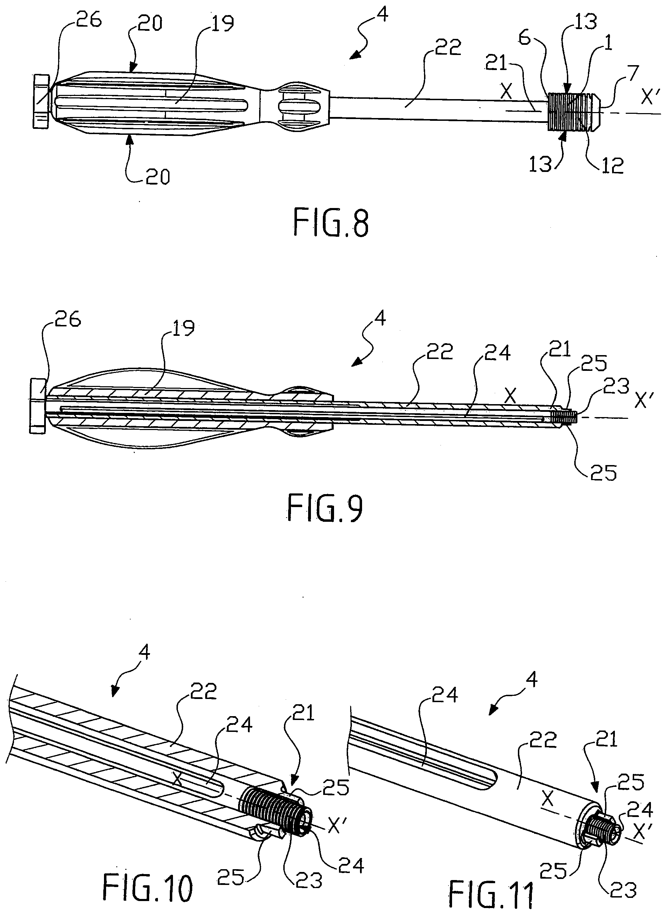

L'implant chirurgical 1 selon l'invention est ainsi conçu pour être inséré entre au moins deux corps osseux 2, 3 du patient, par exemple par impaction dudit implant chirurgical 1 à l'aide d'un outil de mise en place 4 (tel que celui illustré aux figures 8 à 11). Les deux (ou trois, ou quatre, etc.) corps osseux 2, 3 viennent alors de préférence en appui contre l'implant chirurgical 1 interposé entre eux, ce dernier étant conçu pour les maintenir à une distance prédéterminée l'un de l'autre, et/ou pour leur communiquer une déviation angulaire l'un par rapport à l'autre. L'implant chirurgical 1 fonctionne ainsi à la manière d'une cale interposée entre les corps osseux 2, 3, et est ainsi prévu pour être intercalé et coincé entre plusieurs corps osseux 2, 3 du patient afin de leur conférer une position nouvelle. En étant ainsi inséré entre les corps osseux 2, 3 du patient, en contact avec une surface externe respective de ces derniers, l'implant chirurgical 1 permet avantageusement de traiter un symptôme et/ou une pathologie, par exemple osseuse, ou pour le moins liée à la position des os du patient.

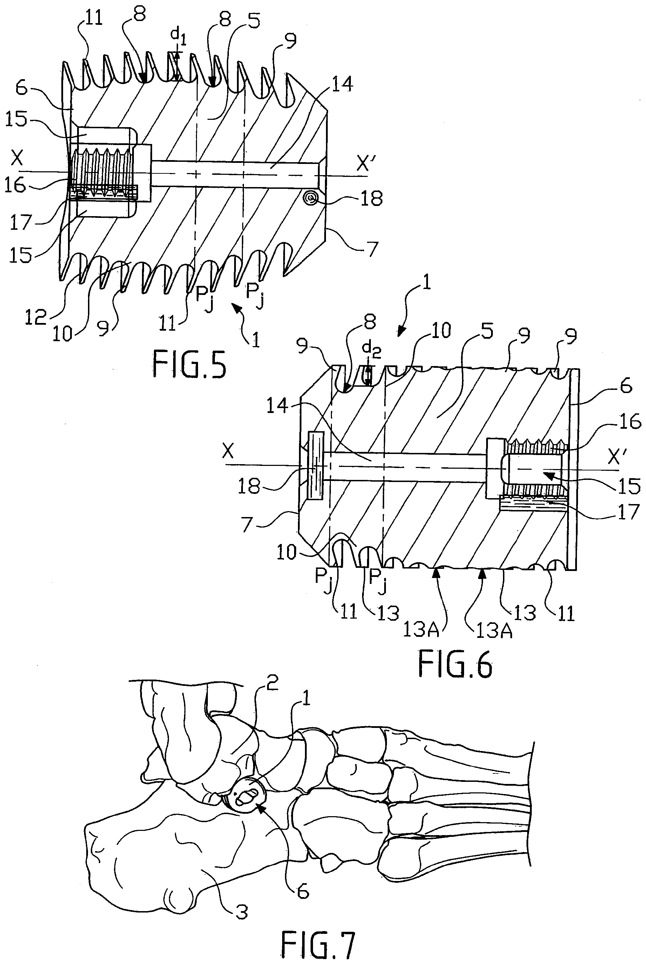

La figure 7 illustre une variante préférentielle de réalisation de l'invention dans laquelle les deux corps osseux 2, 3 sont formés par un talus 2 et un calcanéum 3 d'un pied du patient, ledit implant chirurgical 1 étant destiné à être inséré entre ledit talus 2 et ledit calcanéum 3 afin de traiter, ou de contribuer à traiter, une pathologie du pied plat chez ledit patient. Dans ce cas préférentiel, l'implant chirurgical 1 est inséré dans la cavité du sinus tarsien, afin de modifier l'orientation des deux corps osseux 2, 3 formés en l'espèce par le talus 2 et le calcanéum 3, pour reformer la voûte plantaire naturelle du patient souffrant d'une pathologie de pied plat. Bien entendu, l'invention ne se limite pas à un implant chirurgical 1 destiné à être inséré dans des os du pied du patient ; ledit implant chirurgical 1 pourra par exemple également être conçu et utilisé pour soigner d'autres pathologies, telles qu'une scoliose, en étant inséré entre d'autres corps osseux du corps humain tels que des vertèbres, sans sortir du cadre de l'invention. L'invention pourra également concerner un implant chirurgical 1 insérable entre deux os de la main d'un patient.

L'implant chirurgical 1 de l'invention comprend un corps principal 5 s'étendant le long d'un axe longitudinal X-X' entre une extrémité proximale 6 et une extrémité distale 7 dudit corps principal 5, tel qu'illustré par exemple aux figures 1 à 7. Le corps principal 5 forme une partie centrale de l'implant chirurgical 1 et s'étend en longueur autour de l'axe longitudinal X-X'. Il confère avantageusement à l'implant chirurgical 1 sa forme générale et sa structure mécanique.

L'implant chirurgical 1 est préférentiellement conçu pour être inséré en force, ou par impaction, entre les corps osseux 2, 3, son extrémité distale 7 étant dirigée vers l'avant, vers l'intérieur de la cavité formée par les corps osseux 2, 3. Son extrémité proximale 6 est avantageusement prévue pour recevoir l'outil de mise en place 4, tel que décrit ci-après.

Le corps principal 5 comprend avantageusement une surface latérale 8 entourant l'axe longitudinal X-X' et reliant l'extrémité proximale 6 à l'extrémité distale 7, et contre laquelle les éléments osseux 2, 3 sont destinés à venir en appui. L'implant chirurgical 1 de l'invention est ainsi préférentiellement conçu pour être compressé radialement par les corps osseux 2, 3 entre lesquels il est inséré, afin de repousser ceux-ci à l'écart l'un de l'autre par l'intermédiaire de la surface latérale 8 du corps principal 5.

A cet égard, le corps principal 5 présente de préférence une forme générale de révolution autour de l'axe longitudinal X-X', de manière à épouser, tout au moins au niveau de sa surface latérale 8, la forme des corps osseux 2, 3, contre lesquels il est destiné à venir en appui. Selon la variante préférentielle de l'invention représentée aux figures, le corps principal 5 présente une forme générale de tonneau tronconique, dont l'axe de révolution est l'axe longitudinal X-X', de sorte que l'extrémité proximale 6 forme une grande base du tonneau tronconique et l'extrémité distale 7 forme une petite base du tonneau tronconique. Cette forme particulière de tonneau tronconique, en d'autres termes d'ogive, ou de noisette, confère à l'implant chirurgical 1 la capacité à être adapté à la morphologie osseuse du patient pour pouvoir être inséré de la manière souhaitée, et pour réduire la douleur ressentie par le patient en répartissant les contraintes mécaniques entre la surface des corps osseux 2, 3 et la surface latérale 8. De préférence, cette forme particulière de tonneau tronconique permet également à l'implant chirurgical 1 :
- d'une part de pouvoir pénétrer facilement entre les corps osseux 2, 3 en écartant ces derniers au fur et à mesure de l'insertion dudit implant chirurgical 1,
- d'autre part de rester de lui-même en position en état de coincement entre les deux corps osseux 2, 3, une fois l'insertion de l'implant chirurgical 1 effectuée.

Bien sûr, sans sortir du cadre de l'invention, le corps principal 5 pourra présenter une autre forme générale, par exemple une forme de fuseau, de cône, de sphère ou d'une combinaison de telles formes, ou encore une forme qui n'est pas nécessairement de révolution, mais qui permet d'obtenir des effets techniques similaires de pénétration et de rétention.

Selon l'invention, l'implant chirurgical 1 comprend une pluralité d'ailettes 9, chaque ailette 9 étant pourvue d'un bord de jonction 10 reliant ladite ailette 9 audit corps principal 5, de sorte que lesdites ailettes 9 font saillie dudit corps principal 5 à partir de leur bord de jonction 10 respectif, lequel s'étend sensiblement dans un plan de jonction Pj dont la normale est formée par ledit axe longitudinal X-X'. Des ailerons transversaux à l'axe longitudinal X-X', formés par les ailettes 9, sont ainsi disposés sur une portion de la longueur du corps principal 5, et de préférence sur toute la longueur du corps principal 5, et en particulier sur la surface latérale 8 de ce dernier, tel qu'illustré aux figures. Les ailettes 9 sont avantageusement de géométrie semblable, et sont par exemple homothétiques, isométriques ou homographiques, pour tout ou partie de leur géométrie. Les ailettes 9 ont ainsi avantageusement la même forme, leur taille étant néanmoins adaptée pour suivre le contour du corps principal 5. Les ailettes 9 sont également de préférence orientées de la même manière les unes par rapport aux autres autour de l'axe longitudinal X-X', tel qu'illustré aux figures.

De manière avantageuse, chaque ailette 9 comprend un bord libre 11 et s'étend entre le bord de jonction 10 et le bord libre 11. Chaque ailette 9 est connectée au corps principal 5 par son bord de jonction 10 associé. Le bord libre 11 est destiné à entrer en contact avec les corps osseux 2, 3 du patient. Le bord de jonction 10 de chaque ailette 9 est inclus, dans le plan de jonction Pj virtuel associé, lequel forme un plan transversal à l'axe longitudinal X-X'. De préférence, le bord de jonction 10 est ainsi avantageusement circulaire et centré autour de l'axe X-X'. Sans sortir du cadre de l'invention, le bord de jonction 10 peut avantageusement s'écarter légèrement du plan de jonction Pj de façon proximale et/ou distale à ce dernier, afin de former un contour continu ou non du corps principal 5, lequel contour s'étend en suivant majoritairement le plan de jonction Pj et en s'en écartant localement par endroits, par exemple alternativement en direction de l'extrémité proximale 6 et de l'extrémité distale 7.

Selon une caractéristique de l'invention, chaque ailette 9 présente une souplesse suffisante pour pouvoir être déformée selon une direction centripète et/ou parallèle à l'axe longitudinal X-X' sous l'action de l'un au moins des corps osseux 2, 3 lorsque ledit implant chirurgical 1 est inséré entre lesdits corps osseux 2, 3. Les ailettes 9 sont ainsi conçues pour que la pression des corps osseux 2, 3 soit suffisante, lorsque l'implant chirurgical 1 est interposé entre ces derniers, pour déformer de façon élastique ou plastique une ou plusieurs des ailettes 9, lesquelles se conforment alors sensiblement à la forme imposée par les corps osseux 2, 3, et répartissent de cette manière sensiblement la pression de contact entre les corps osseux 2, 3 et l'implant chirurgical 1, afin en particulier de réduire la douleur ressentie par le patient liée à la présence de cet implant chirurgical 1. De préférence, les ailettes 9 sont conçues pour se déformer sous l'action des corps osseux 2, 3 en fléchissant, en se rabattant le long du corps principal 5 ou en s'écrasant sur elles-mêmes. Lorsque l'implant chirurgical est inséré, les corps osseux 2, 3 et notamment leur surface externe, ne sont avantageusement eux-mêmes pas déformés, ou de manière infime en comparaison de la déformation subie par les ailettes 9.

Par ailleurs, les ailettes 9 sont avantageusement inclinées par rapport à l'axe longitudinal X-X' de manière à faire saillie du corps principal 5 en direction de l'extrémité proximale 6, avec un angle d'inclinaison compris entre 45 et 90° par rapport à l'axe longitudinal X-X'. Cette inclinaison contribue avantageusement à la souplesse des ailettes 9, lesquelles auront une tendance naturelle à fléchir sur sollicitation des corps osseux 2, 3 en direction de l'extrémité proximale 6. Également, cette inclinaison permet de préférence aux ailettes 9 de contribuer à la rétention de l'implant chirurgical 1 entre les corps osseux 2, 3. Sans sortir du cadre de l'invention, chaque ailette 9 peut avantageusement s'étendre de façon sensiblement radiale à l'axe longitudinal X-X'.

De plus, les ailettes 9 sont avantageusement sensiblement parallèles entre elles, et espacées régulièrement l'une de l'autre d'une distance comprise entre 0,2 mm et 5 mm, de préférence 1,20 mm. Leur parallélisme et leur espacement permet auxdites ailettes 9 de se déformer facilement pour s'adapter à la forme des corps osseux 2, 3 et répartir les contraintes de pression. Le bord de jonction 10 est préférentiellement formé par un congé, ou un arrondi, de manière à ce que chaque gorge séparant deux ailettes 9 forme un sillon sensiblement dénué d'arête, afin notamment de limiter les risques de rupture (par concentration de contraintes) des ailettes 9 par leur bord de jonction 10 respectif, lors de la sollicitation mécanique desdites ailettes 9 par les corps osseux 2, 3.

Dans la variante préférentielle de l'invention représentée aux figures, au moins l'une desdites ailettes 9, sinon la majorité des ailettes 9, sinon toutes les ailettes 9, s'étend(ent) sur au moins la majorité, sinon la totalité, du périmètre du corps principal 5 de manière à entourer l'axe longitudinal X-X', et forment ainsi des côtes circulaires transversales séparées par des sillons transversaux à l'axe longitudinal X-X', avantageusement de largeur égale deux à deux. Les ailettes 9 encerclent avantageusement complètement le corps principal 5, sur toute sa circonférence. Sans sortir du cadre de l'invention, les ailettes 9 pourront toutefois être discontinues et ne s'étendre chacune que sur une ou plusieurs portion(s) du périmètre du corps principal 5.

Selon la variante préférée représentée aux figures, les ailettes 9 s'étendent chacune de façon sensiblement conique par rapport à l'axe longitudinal X-X'.

Afin de garantir leur souplesse tout en limitant sensiblement tout risque de dégradation ou de lésion de la surface des corps osseux 2, 3, les ailettes 9 ont préférentiellement une épaisseur supérieure à 0,10 mm et inférieure à 2 mm de manière à être souples sans être coupantes, l'implant chirurgical 1 et en particulier lesdites ailettes 9, étant réalisés avantageusement dans un matériau présentant un module de Young compris entre 0,45 GPa et 15 GPa, de préférence inférieur à 10 GPa, c'est-à-dire inférieur au module de Young des os corticaux du patient, ou des cartilages du patient, et supérieur au module de Young des os spongieux du patient. A cet effet, de façon préférentielle, l'implant chirurgical 1 est réalisé en polymère biocompatible, préférentiellement du polyétheréthercétone (PEEK). L'implant chirurgical 1 forme avantageusement une unique pièce monobloc, incluant en particulier les ailettes 9, réalisée par exemple par moulage, injection, ou usinage d'une pièce brute monobloc, et est ainsi relativement facile et peu coûteux à fabriquer. D'autres matériaux pourront être envisagés sans sortir du cadre de l'invention, tels qu'un métal biocompatible comme le titane, ou un matériau bio-résorbable.

Le corps principal 5 présente de préférence également une certaine souplesse, qui est inférieure à celle des ailettes 9, de manière à pouvoir se déformer sous l'action de l'un au moins des corps osseux 2, 3 lorsque ledit implant chirurgical 1 est inséré entre lesdits corps osseux 2, 3, dans le cas où au moins l'une des ailettes 9 est complètement déformée.

De préférence, l'implant chirurgical 1, lorsqu'il est inséré entre les corps osseux 2, 3 du patient, se déforme en deux temps afin de s'adapter à la morphologie desdits corps osseux 2, 3 :
- un premier temps au cours duquel les ailettes 9 sont déformées,
- un deuxième temps au cours duquel le corps principal 5 est déformé, après déformation des ailettes 9.

De manière avantageuse, chaque ailette 9 présente au moins :
- une portion de largeur constante 12, le long de laquelle le bord libre 11 et le bord de jonction 10 sont séparés par une première distance d₁ sensiblement constante le long de ladite portion de largeur constante 12, et
- une portion de méplat 13, le long de laquelle le bord libre 11 et le bord de jonction 10 sont séparés par une deuxième distance d₂ inférieure à la première distance d₁.

Dans la variante préférentielle illustrée aux figures, le corps principal 5 forme préférentiellement un solide de révolution autour de l'axe longitudinal X-X', de sorte que le bord de jonction 10 forme un cercle continu ou discontinu, l'axe longitudinal X-X' passant par le centre de ce cercle. Dans ce cas préférentiel, le long de la portion de largeur constante 12, le bord libre 11 forme avantageusement un arc de cercle concentrique avec le cercle formé par le bord de jonction 10, de sorte que le bord libre 11 et le bord de jonction 10 forment deux courbes parallèles le long de ladite portion de largeur constante 12.

Le long de la portion de méplat 13, le bord libre 11 est préférentiellement rectiligne de manière à former une portion rectiligne 13A. La deuxième distance d₂ séparant la portion rectiligne 13A du bord libre 11 et le bord de jonction 10 le long de la portion de méplat 13 présente un minimum, qui peut être nul (tel qu'illustré aux figures), le bord libre 11 et le bord de jonction 10 étant dès lors confondus à cet endroit.

De préférence, la distance d₁, formant en l'espèce la profondeur maximale de la gorge inter-ailettes 9, mesure environ 2,8 mm.

Dans la variante préférée représentée aux figures, chaque ailette 9 présente au moins deux portions de méplat 13, le long de chacune desquelles le bord libre 11 présente une portion rectiligne 13A pour former une portion de méplat 13 rectiligne, les portions de méplat 13 étant réparties régulièrement autour de l'axe longitudinal X-X', de préférence de façon symétrique l'une par rapport à l'autre, par exemple de manière à ce que les portions rectilignes du bord libre 11 soient parallèles entre elles. Chaque ailette 9 est ainsi avantageusement formée, autour de l'axe longitudinal X-X', d'une alternance de portions de méplat 13 et de portions de largeur constante adjacentes.

Les deux portions de méplat 13 ainsi disposées permettent :
- d'une part à l'implant chirurgical d'être particulièrement adapté à la morphologie des corps osseux 2, 3 entre lesquels il est destiné à être inséré, en particulier à la morphologie de la cavité ménagée entre le calcanéum 3 et le talus 2 du patient,
- d'autre part à favoriser la déformation des ailettes 9 par les corps osseux 2, 3 en répartissant, au sein desdites ailettes 9, les contraintes que lesdits corps osseux 2, 3 imposent auxdites ailettes 9 lorsque l'implant chirurgical 1 est inséré.

De préférence, et tel qu'illustré aux figures, l'implant chirurgical 1 comprend en outre une ouverture traversante 14, c'est-à-dire une canule, ménagée dans le corps principal 5 le long de l'axe longitudinal X-X', depuis l'extrémité proximale 6 jusqu'à l'extrémité distale 7. Lorsque l'implant chirurgical 1 est inséré dans le corps du patient, l'ouverture traversante 14 permet notamment au chirurgien, ou utilisateur, de pouvoir effectuer une observation à travers ledit implant chirurgical 1, préférentiellement en étant placé lui-même du côté de l'extrémité proximale 6. L'ouverture traversante 14 permet également préférentiellement de faire passer un produit ou un tube à travers l'implant, par exemple pour insérer un produit ostéo-inducteur dans la cavité du sinus tarsien.

L'ouverture traversante 14 est préférentiellement conçue pour accueillir l'outil de mise en place 4 du côté de l'extrémité proximale 6, et inclut à cet effet au moins une encoche 15 d'interface destinée à coopérer avec un outil de mise en place 4 de l'implant chirurgical 1, afin que ledit outil de mise en place 4 puisse entraîner en rotation l'implant chirurgical 1 lorsqu'il est inséré dans l'ouverture traversante 14 en coopération avec ladite encoche 15. L'encoche 15 est de préférence ménagée à partir de l'extrémité proximale 6, et s'étend longitudinalement le long de l'ouverture traversante 14 jusqu'à environ un tiers de la longueur de l'implant chirurgical 1. L'outil de mise en place 4 peut ainsi coopérer avec l'encoche 15 pour fournir par exemple un moment de rotation autour de l'axe longitudinal X-X' audit implant 1, à la manière d'un tournevis. Bien entendu, sans sortir du cadre de l'invention, l'ouverture traversante 14 peut accueillir une pluralité d'encoches 15 adaptées en l'espèce à la forme de l'outil de mise en place 4. En particulier, tel qu'illustré aux figures 5 et 6, l'ouverture traversante inclut deux encoches 15 disposées de part et d'autre de l'axe longitudinal X-X', symétriquement, de manière à former une ouverture oblongue ou allongée du côté de l'extrémité proximale 6, et dont le grand diamètre est sensiblement parallèle à la portion rectiligne 13A du bord libre 11, de manière à ce que l'implant chirurgical 1 présente une forme générale symétrique. L'outil de mise en place 4 comporte de préférence une tête 21 pourvue de deux ergots 25 conçus pour être insérés dans les encoches 15 afin de solidariser à rotation autour de l'axe longitudinal X-X' ledit implant chirurgical 1 avec l'outil de mise en place 4.

L'ouverture traversante 14 inclut avantageusement un filetage intérieur 16 s'étendant à partir de l'extrémité proximale 6, qui permet notamment à un élément de vissage 23 de l'outil de mise en place 4 de coopérer avec ledit filetage intérieur 16, afin de permettre la préhension de l'implant chirurgical par l'outil de mise en place 4, par l'intermédiaire de la coopération dudit filetage intérieur 16 avec ledit élément de vissage 23.

De préférence, l'implant chirurgical 1 formé d'une matière invisible à la radiographie, c'est-à-dire radio-transparente, par exemple du polyétheréthercétone ou une autre matière plastique biocompatible.

Selon la variante préférée de l'invention représentée aux figures, l'implant chirurgical 1 comprend en outre :
- un élément radio-repère proximal 17 disposé au sein du corps principal 5 au voisinage de l'extrémité proximale 6, et
- un élément radio-repère distal 18 disposé au sein du corps principal 5 au voisinage de l'extrémité distale 7.

Dans ce cas préférentiel, l'implant chirurgical 1 est ainsi invisible à la radiographie, excepté les éléments radio-repères distal 18 et proximal 17. Ainsi, un chirurgien (ou un utilisateur) peut avantageusement vérifier le bon positionnement de l'implant chirurgical 1 dans le corps du patient à l'aide des éléments radio-repères distal 18 et proximal 17, la masse de l'implant chirurgical 1 n'obstruant pas la radiographie, de sorte que les éléments osseux 2, 3 sont visibles au travers dudit implant chirurgical 1 sur la radiographie. Le chirurgien, ou utilisateur, peut donc préférentiellement contrôler l'état de ces derniers, ainsi que l'avancement du traitement de la pathologie de manière particulièrement aisée.

Lesdits éléments radio-repères proximal et distal sont de préférence des fils radio-repère, par exemple métalliques, lesquels sont disposés au sein du corps principal 5 avec une orientation distincte l'une de l'autre. De cette manière avantageuse, le chirurgien, ou utilisateur, peut évaluer à la fois la position et l'orientation de l'implant chirurgical 1 en examinant une radiographie de la zone d'implantation de l'implant chirurgical 1 dans le corps du patient, les deux éléments radio-repères distal 18 et proximal 17 étant visibles sur ladite radiographie, et en particulier leur orientation relative, alors que le reste de l'implant chirurgical 1 reste invisible ou transparent.

Dans ce cas préférentiel, l'implant chirurgical 1 est ainsi avantageusement formé par une pièce monobloc incluant notamment le corps principal 5 et les ailettes 9, résultant par exemple de l'usinage d'une pièce brute en polyétheréthercétone, ou d'un moulage ou d'une injection de polyétheréthercétone dans un moule, sur lequel sont rapportés les éléments radio-repères distal 18 et proximal 17, métalliques, dans des perçages de la pièce monobloc (tel qu'illustré aux figures 3 à 6).

L'invention concerne également en tant que tel un outil de mise en place 4 d'un implant chirurgical 1 tel que décrit ci-avant, tel qu'illustré en particulier aux figures 8 à 11. L'outil de mise en place 4 forme avantageusement l'outil de mise en place 4 décrit ci-avant Selon l'invention, l'outil de mise en place 4 comprend :
- une tête 21 conçue pour coopérer à rotation avec l'implant chirurgical 1 autour de l'axe longitudinal X-X' de ce dernier, et
- une poignée de préhension 19 par l'intermédiaire de laquelle un utilisateur, en particulier chirurgien, peut saisir ledit outil de mise en place 4, ladite poignée comportant un témoin visuel 20 de l'orientation de l'implant chirurgical 1 autour de son axe longitudinal X-X' lorsque ledit outil de mise en place 4 est en coopération avec ledit implant chirurgical 1.

L'outil de mise en place 4 présente avantageusement l'allure générale d'un tournevis, et s'étend le long d'un axe d'extension destiné à correspondre avec l'axe longitudinal X-X' de l'implant chirurgical 1, entre la poignée de préhension 19 et la tête 21, la tête 21 étant reliée de façon solidaire à la poignée de préhension 19 par l'intermédiaire d'une tige 22. Dans la variante préférentielle de l'outil de mise en place 4 représentée à la figure 9, la tige 22 traverse de part en part la poignée de préhension 19.

Une canule longitudinale 24 est préférentiellement ménagée le long de la poignée de préhension 19, de la tête 21 et/ou de la tige 22, ladite canule longitudinale 24 étant conçue pour se placer en regard avec l'ouverture traversante 14 de l'implant chirurgical 1 lorsque ce dernier est connecté à la tête 21, de manière à ce que la canule longitudinale 24 et l'ouverture traversante 14 forment un canal continu (ou au moins communiquent) reliant l'extrémité distale 7 de l'implant chirurgical 1 à l'outil de mise en place 4, en particulier à l'extrémité libre de la poignée de préhension 19. De cette manière, l'utilisateur peut par exemple introduire un fluide ou un tube dans le corps du patient par l'intermédiaire et au travers de l'outil de préhension 19 et l'implant chirurgical 1.

De manière préférentielle, le témoin visuel 20 est formé par un ou deux méplat(s) ménagés dans le manche de préhension 19, destinés à être alignés avec les portions de méplat 13 de l'implant chirurgical 1. Dans le cas de cette variante préférentielle représentée à la figure 8, l'outil de mise en place 4 est conçu pour que, lorsque l'implant chirurgical 1 est connecté avec ledit outil de mise en place 4 par l'intermédiaire de l'ouverture traversante 14, les méplats formant le témoin visuel 20 sont alignés et parallèles avec les portions de méplat 13 de l'implant chirurgical 1, tel que cela est visible en particulier à la figure 8. En l'espèce, le manche de préhension 19 présente une forme générale qui est de nature à imiter la forme générale de l'implant chirurgical 1, le manche de préhension 19 étant conçu pour présenter la même orientation autour de l'axe longitudinal X-X' que l'implant chirurgical 1, de sorte que l'utilisateur (ie. le chirurgien) peut connaître l'orientation dudit implant chirurgical 1 de manière intuitive, en observant simplement l'orientation du manche de préhension 19. Lors de la mise en place de l'implant chirurgical 1 sur la tête 21, la coopération entre les encoches 15 et les ergots 25 permet de positionner et d'orienter l'implant chirurgical 1 sur l'outil de mise en place 4 de manière à ces que les portions de méplat 13 et les témoins visuels 20 soient alignés et parallèles.

Tel qu'illustré en particulier aux figures 9 à 11, l'élément de vissage 23 fait avantageusement saillie de l'extrémité de la tête 21 de l'outil de mise en place 4, entre les deux ergots 25. L'élément de vissage 23 est préférentiellement monté à rotation dans la tige 22 autour de l'axe longitudinal X-X', et optionnellement à translation le long de l'axe longitudinal X-X'. L'élément de vissage 23 est entraîné de préférence par une molette de vissage 26 de l'outil de mise en place 4 traversant de part en part ce dernier et en particulier la tige 22. La molette de vissage 26 est actionnable par exemple depuis l'extrémité de la poignée de préhension 19, tel qu'illustré aux figures 8 et 9. La canule longitudinale 24 est de préférence ménagée dans la molette de vissage 26, dans le sens de la longueur, pour déboucher à l'extrémité de l'élément de vissage 23.

L'invention concerne en outre, en tant que tel, un kit chirurgical incluant :
- un implant chirurgical 1 tel que décrit ci-avant,
- un outil de mise en place 4 de l'implant chirurgical 1, ledit outil de mise en place 4 étant par exemple conforme à celui décrit ci-avant.

Ainsi, de façon préférentielle, ledit outil de mise en place 4 comprend :
- une tête (21) conçue pour coopérer à rotation avec l'implant chirurgical 1 autour de l'axe longitudinal X-X' de ce dernier, et
- une poignée de préhension 19 par l'intermédiaire de laquelle un utilisateur peut saisir ledit outil de mise en place 4, ladite poignée comportant un témoin visuel 20 de l'orientation de l'implant chirurgical 1 autour de son axe longitudinal X-X' lorsque ledit outil de mise en place 4 est en coopération avec ledit implant chirurgical 1.

L'invention concerne en outre en tant que tel un procédé de fabrication d'un implant chirurgical 1 tel que décrit précédemment.

Le procédé de fabrication de l'invention peut être mené de deux façons alternatives et distinctes, et comprend en l'espèce :
- soit une étape de moulage d'une pièce finie monobloc destinée à former l'implant chirurgical 1,
- soit une étape d'usinage d'une pièce brute monobloc pour former une pièce finie monobloc destinée à former elle-même l'implant chirurgical 1.

Il est ainsi possible de réaliser l'implant chirurgical 1 à l'aide d'une unique étape de moulage, dans le cadre par exemple d'une production en grande série d'implants chirurgicaux de ce type.

Alternativement, dans le cas par exemple d'une production en petite série, l'implant chirurgical 1 peut être réalisé par conformation d'une pièce brute d'un seul tenant, à l'aide par exemple d'une machine d'usinage à commande numérique. La pièce brute a avantageusement été réalisée au préalable par moulage.

De manière avantageuse, la pièce brute monobloc, destinée à l'usinage, est en polymère, de préférence en polyétheréthercétone (PEEK), de manière à former, après usinage, un implant chirurgical 1 monobloc, intégralement en polymère.

Préférentiellement, la pièce finie monobloc réalisée par moulage ou par usinage, est en polymère, de préférence en polyétheréthercétone (PEEK).

Le procédé de fabrication comporte préférentiellement une étape supplémentaire dans laquelle on ménage deux logements dans la pièce brute, ou dans le corps principal 5 de l'implant chirurgical 1, afin d'y insérer un élément radio-repère proximal 17 et un élément radio-repère distal 18 (tel qu'illustré aux figures).

De façon subsidiaire, l'invention pourra concerner en tant que tel un procédé de mise en place d'un implant chirurgical 1 dans le corps d'un patient à l'aide d'un outil de mise en place 4, ledit procédé de mise en place étant susceptible de faire l'objet d'une protection en tant que tel. Dans le cadre de ce procédé de mise en place, l'implant chirurgical 1 et l'outil de mise en place 4 sont préférentiellement conformes à la description qui précède.

Ce procédé de mise en place de l'implant 1 comporte avantageusement une ou plusieurs des étapes suivantes.

On solidarise l'implant chirurgical 1 à l'outil de mise en place 4, de préférence en faisant interagir la tête 21 de l'outil de mise en place 4 avec l'ouverture traversante 14 de l'implant chirurgical. Lors de la solidarisation, on visse préférentiellement l'élément de vissage 23 de l'outil de mise en place 4 avec le filetage intérieur 16 de l'implant chirurgical 1 en actionnant la molette de vissage 26, et on fait coopérer au moins une encoche 15 avec la tête 21 de l'outil de mise en place 4 de façon à ce que ce dernier soit en mesure d'entraîner en rotation ledit implant chirurgical autour de son axe longitudinal X-X' par rotation de la poignée de préhension 19.

On insère de préférence l'implant chirurgical 1 entre les corps osseux 2, 3 du patient souhaités en tenant ledit implant chirurgical par l'intermédiaire de l'outil de mise en place 4. L'insertion s'effectue de préférence en force, ou par impaction, de l'implant chirurgical 1 de manière à coincer ce dernier entre les corps osseux 2, 3, et à ce que ledit implant chirurgical 1 soit en mesure de modifier l'orientation, et/ou la position relative, des corps osseux 2, 3 à la manière d'une cale.

On règle avantageusement l'orientation de l'implant chirurgical 1 autour de l'axe longitudinal X-X' afin d'ajuster sa forme générale à la forme de la cavité ménagée entre les corps osseux 2, 3 par rotation autour dudit axe longitudinal X-X' de l'outil de mise en place 4.

De préférence, lors de son insertion entre les corps osseux 2, 3, les ailettes 9 se déforment afin d'épouser sensiblement la forme morphologique desdits corps osseux 2, 3. Les ailettes 9 étant déformées de façon plus ou moins complète, le corps principal 5 se déforme également légèrement de façon élastique.

On désolidarise l'implant chirurgical 1 de l'outil de mise en place 4, avantageusement en dévissant l'élément de vissage 23 du filetage intérieur 16, et en séparant la tête 21 de l'ouverture traversante 14.

### POSSIBILITE D'APPLICATION INDUSTRIELLE

L'invention trouve son application industrielle dans la conception, la mise en oeuvre et la fabrication, notamment à l'aide des procédés de fabrication de moulage ou d'usinage décrits précédemment et utilisables de manière industrielle, d'implants chirurgicaux de correction du positionnement ou de l'orientation mutuel(le) des os d'un patient.

## Revendications

1. Implant chirurgical (1) de correction du positionnement ou de l'orientation mutuel(le) des os d'un patient conçu pour être inséré entre au moins deux corps osseux (2, 3) dudit patient, ledit implant chirurgical (1) comprenant :
- un corps principal (5) s'étendant le long d'un axe longitudinal (X-X') entre une extrémité proximale (6) et une extrémité distale (7) dudit corps principal (5),
- une pluralité d'ailettes (9), chaque ailette (9) étant pourvue d'un bord de jonction (10) reliant ladite ailette (9) audit corps principal (5), de sorte que lesdites ailettes (9) font saillie dudit corps principal (5) à partir de leur bord de jonction (10) respectif, lequel s'étend sensiblement dans un plan de jonction (Pj) dont la normale est formée par ledit axe longitudinal (X-X'), chaque ailette (9) comprenant un bord libre (11) et s'étendant entre le bord de jonction (10) et le bord libre (11),
l'implant chirurgical (1) étant **caractérisé en ce que** chaque ailette (9) présente une souplesse suffisante pour pouvoir être déformée selon une direction centripète et/ou parallèle à l'axe longitudinal (X-X') sous l'action de l'un au moins des corps osseux (2, 3) lorsque ledit implant chirurgical (1) est inséré entre lesdits corps osseux (2, 3), et **en ce que** chaque ailette (9) présente au moins :
- une portion de largeur constante (12), le long de laquelle le bord libre (11) et le bord de jonction (10) sont séparés par une première distance (d1) sensiblement constante le long de ladite portion de largeur constante (12), et,
- une portion de méplat (13), le long de laquelle le bord libre (11) et le bord de jonction (10) sont séparés par une deuxième distance (d2) inférieure à la première distance (d1).

2. Implant chirurgical (1) selon la revendication précédente, **caractérisé en ce qu'**il forme une unique pièce monobloc, réalisée par exemple par moulage, injection, ou usinage d'une pièce brute monobloc.

3. Implant chirurgical (1) selon la revendication précédente, **caractérisé en ce que** le corps principal (5) présente une souplesse inférieure à celle des ailettes (9), de manière à pouvoir se déformer sous l'action de l'un au moins des corps osseux (2, 3) lorsque ledit implant chirurgical (1) est inséré entre lesdits corps osseux (2, 3) dans le cas où au moins l'une des ailettes (9) est complètement déformée.

4. Implant chirurgical (1) selon l'une quelconque des revendications précédentes, **caractérisé en ce qu'**au moins l'une desdites ailettes (9) s'étend sur au moins la majorité, sinon la totalité, du périmètre du corps principal (5) de manière à entourer l'axe longitudinal (X-X').

5. Implant chirurgical (1) selon l'une quelconque des revendications précédentes, **caractérisé en ce que** les ailettes (9) sont inclinées par rapport à l'axe longitudinal (X-X') de manière à faire saillie du corps principal (5) en direction de l'extrémité proximale (6), avec un angle d'inclinaison compris entre 45 et 90° par rapport à l'axe longitudinal (X-X').

6. Implant chirurgical (1) selon l'une quelconque des revendications précédentes, **caractérisé en ce que** chaque ailette (9) présente au moins deux portions de méplat (13), le long de chacune desquelles le bord libre (11) présente une portion rectiligne (13A) pour former une portion de méplat (13) rectiligne, les portions de méplat (13) étant réparties régulièrement autour de l'axe longitudinal (X-X'), de préférence de façon symétrique l'une par rapport à l'autre.

7. Implant chirurgical (1) selon l'une quelconque des revendications 1, ou 3 à 6, **caractérisé en ce qu'**il comprend en outre :
- un élément radio-repère proximal (17) disposé au sein du corps principal (5) au voisinage de l'extrémité proximale (6), et
- un élément radio-repère distal (18) disposé au sein du corps principal (5) au voisinage de l'extrémité distale (7),
lesdits éléments radio-repères proximal et distal étant de préférence des fils radio-repère, lesquels sont disposés au sein du corps principal (5) avec une orientation distincte l'une de l'autre.

8. Implant chirurgical (1) selon l'une quelconque des revendications précédentes, **caractérisé en ce qu'**il comprend en outre une ouverture traversante (14) ménagée dans le corps principal (5) le long de l'axe longitudinal (X-X').

9. Implant chirurgical (1) selon la revendication précédente, **caractérisé en ce que** l'ouverture traversante (14) inclut un filetage intérieur (16) s'étendant à partir de l'extrémité proximale (6).

10. Implant chirurgical (1) selon l'une quelconque des revendications 8 ou 9, **caractérisé en ce que** l'ouverture traversante (14) inclut au moins une encoche (15) d'interface destinée à coopérer avec un outil de mise en place (4) de l'implant chirurgical (1), afin que ledit outil de mise en place (4) puisse entraîner en rotation l'implant chirurgical (1) lorsqu'il est inséré dans l'ouverture traversante (14) en coopération avec ladite encoche (15).

11. Implant chirurgical (1) selon l'une quelconque des revendications précédentes, **caractérisé en ce qu'**il est un implant de traitement d'une pathologie du pied plat chez ledit patient.

12. Kit chirurgical incluant :
- un implant chirurgical (1) selon l'une quelconque des revendications 1 à 11,
- un outil de mise en place (4) de l'implant chirurgical (1).

13. Kit chirurgical selon la revendication 12 **caractérisée en ce que** l'outil de mise en place (4) comprend :
- une tête (21) conçue pour coopérer à rotation avec l'implant chirurgical (1) autour de l'axe longitudinal (X-X') de ce dernier, et
- une poignée de préhension (19) par l'intermédiaire de laquelle un utilisateur peut saisir ledit outil de mise en place (4), ladite poignée comportant un témoin visuel (20) de l'orientation de l'implant chirurgical (1) autour de son axe longitudinal (X-X') lorsque ledit outil de mise en place (4) est en coopération avec ledit implant chirurgical (1).

14. Procédé de fabrication d'un implant chirurgical (1) selon l'une quelconque des revendications 1 à 11, **caractérisé en ce qu'**il comprend :
- soit une étape de moulage d'une pièce finie monobloc destinée à former l'implant chirurgical (1),
- soit une étape d'usinage d'une pièce brute monobloc pour former une pièce finie monobloc destinée à former elle-même l'implant chirurgical (1),
laquelle pièce brute monobloc et/ou la pièce finie monobloc est de préférence en polymère, de préférence encore en polyétheréthercétone (PEEK).

## Patentansprüche

1. Chirurgisches Implantat (1) zum Korrigieren der gegenseitigen Positionierung oder Ausrichtung der Knochen eines Patienten, dazu ausgelegt, zwischen mindestens zwei Knochenkörper (2, 3) des Patienten eingesetzt zu werden, wobei das chirurgische Implantat (1) umfasst:
- einen Hauptkörper (5), der sich entlang einer Längsachse (X-X') zwischen einem proximalen Ende (6) und einem distalen Ende (7) des Hauptkörpers (5) erstreckt,
- eine Vielzahl von Rippen (9), wobei jede Rippe (9) mit einer Verbindungskante (10) versehen ist, die die Rippe (9) mit dem Hauptkörper (5) verbindet, sodass die Rippen (9) von ihrer jeweiligen Verbindungskante (10) aus dem Hauptkörper (5) hervorstehen, wobei sich die Verbindungskante im Wesentlichen in einer Verbindungsebene (Pj) erstreckt, deren Normale durch die Längsachse (X-X') gebildet wird, wobei jede Rippe (9) eine freie Kante (11) umfasst und sich zwischen der Verbindungskante (10) und der freien Kante (11) erstreckt,
wobei das chirurgische Implantat (1) **dadurch gekennzeichnet ist, dass** jede Rippe (9) eine ausreichende Flexibilität aufweist, um gemäß einer zentripetalen Richtung und/oder parallel zur Längsachse (X-X') unter der Einwirkung von mindestens einem dieser Knochenkörper (2, 3) verformt zu werden, wenn das chirurgische Implantat (1) zwischen diesen Knochenkörpern (2, 3) eingesetzt ist, und dadurch, dass jede Rippe (9) mindestens:
- einen Abschnitt konstanter Breite (12), entlang dem die freie Kante (11) und die Verbindungskante (10) durch einen ersten im Wesentlichen konstanten Abstand (d1) entlang des Abschnitts konstanter Breite (12) getrennt sind, und
- einen flachen Abschnitt (13), entlang dem die freie Kante (11) und die Verbindungskante (10) durch einen zweiten Abstand (d2) getrennt sind, der kleiner als der erste Abstand (d1) ist.

2. Chirurgisches Implantat (1) nach dem vorhergehenden Anspruch, **dadurch gekennzeichnet, dass** es ein einziges, einteiliges Stück bildet, das beispielsweise durch Formen, Spritzgießen oder Bearbeiten eines einteiligen Rohlings ausgeführt wird.

3. Chirurgisches Implantat (1) nach dem vorhergehenden Anspruch, **dadurch gekennzeichnet, dass** der Hauptkörper (5) eine geringere Flexibilität als die der Rippen (9) aufweist, sodass er sich unter der Einwirkung von mindestens einem der Knochenkörper (2, 3) verformen kann, wenn das chirurgische Implantat (1) zwischen den Knochenkörpern (2, 3) eingesetzt ist, falls wenigstens eine der Rippen (9) vollständig verformt ist.

4. Chirurgisches Implantat (1) nach einem der vorhergehenden Ansprüche, **dadurch gekennzeichnet, dass** wenigstens eine der Rippen (9) sich über mindestens den größten Teil oder auch den gesamten Umfang des Hauptkörpers (5) erstreckt, sodass sie die Längsachse (X-X') umgibt.

5. Chirurgisches Implantat (1) nach einem der vorhergehenden Ansprüche, **dadurch gekennzeichnet, dass** die Rippen (9) bezogen auf die Längsachse (X-X') so geneigt sind, dass sie vom Hauptkörper (5) in Richtung des proximalen Endes (6) mit einem Neigungswinkel zwischen 45 und 90° in Bezug auf die Längsachse (X-X') hervorstehen.

6. Chirurgisches Implantat (1) nach einem der vorhergehenden Ansprüche, **dadurch gekennzeichnet, dass** jede Rippe (9) mindestens zwei abgeflachte Abschnitte (13) aufweist, entlang derer die freie Kante (11) jeweils einen geradlinigen Abschnitt (13A) aufweist, um einen geradlinigen abgeflachten Abschnitt (13) zu bilden, wobei die abgeflachten Abschnitte (13) gleichmäßig um die Längsachse (X-X') verteilt sind, vorzugsweise symmetrisch zueinander.

7. Chirurgisches Implantat (1) nach einem der Ansprüche 1 oder 3 bis 6, **dadurch gekennzeichnet, dass** es ferner Folgendes umfasst:
- ein proximales Radiomarkierungselement (17), das innerhalb des Hauptkörpers (5) in der Nähe des proximalen Endes (6) angeordnet ist, und
- ein distales Radiomarkierungselement (18), das innerhalb des Hauptkörpers (5) in der Nähe des distalen Endes (7) angeordnet ist,
wobei das proximale und das distale Radiomarkierungselement vorzugsweise Radiomarkierungsdrähte sind, die innerhalb des Hauptkörpers (5) mit einer unterschiedlichen Ausrichtung zueinander angeordnet sind.

8. Chirurgisches Implantat (1) nach einem der vorhergehenden Ansprüche, **dadurch gekennzeichnet, dass** es ferner eine Durchgangsöffnung (14) umfasst, die im Hauptkörper (5) entlang der Längsachse (X-X') vorgesehen ist.

9. Chirurgisches Implantat (1) nach dem vorhergehenden Anspruch, **dadurch gekennzeichnet, dass** die Durchgangsöffnung (14) ein Innengewinde (16) einschließt, das sich vom proximalen Ende (6) aus erstreckt.

10. Chirurgisches Implantat (1) nach einem der Ansprüche 8 oder 9, **dadurch gekennzeichnet, dass** die Durchgangsöffnung (14) mindestens eine Grenzflächenaussparung (15) einschließt, die dazu bestimmt ist, mit einem Werkzeug zum Einsetzen (4) des chirurgischen Implantats (1) zusammenzuwirken, damit das Werkzeug zum Einsetzen (4) das chirurgische Implantat (1) im Zusammenwirken mit der Aussparung (15) drehen kann, wenn es in die Durchgangsöffnung (14) eingesetzt wird.

11. Chirurgisches Implantat (1) nach einem der vorhergehenden Ansprüche, **dadurch gekennzeichnet, dass** es sich um ein Implantat zur Behandlung einer Pathologie des Plattfußes bei dem Patienten handelt.

12. Chirurgisches Kit, einschließlich:
- eines chirurgischen Implantats (1) nach einem der Ansprüche 1 bis 11,
- eines Werkzeugs zum Einsetzen (4) des chirurgischen Implantats (1).

13. Chirurgisches Kit nach Anspruch 12, **dadurch gekennzeichnet, dass** das Werkzeug zum Einsetzen (4) Folgendes umfasst:
- einen Kopf (21), der ausgelegt ist, um drehbar mit dem chirurgischen Implantat (1) um dessen Längsachse (X-X') zusammenzuwirken, und
- einen Greifgriff (19), durch den ein Benutzer das Werkzeug zum Einsetzen (4) greifen kann, wobei der Griff eine visuelle Anzeige (20) der Orientierung des chirurgischen Implantats (1) um seine Längsachse (X-X') aufweist, wenn das Werkzeug zum Einsetzen (4) mit dem chirurgischen Implantat (1) zusammenwirkt.

14. Verfahren zur Herstellung eines chirurgischen Implantats (1) nach einem der Ansprüche 1 bis 11, **dadurch gekennzeichnet, dass** es Folgendes umfasst:
- entweder einen Schritt des Formens eines einteiligen Fertigteils, das das chirurgische Implantat (1) bilden soll,
- oder einen Schritt des Bearbeitens eines einteiligen Rohlings, um ein einteiliges Fertigteil zu bilden, das wiederum das chirurgische Implantat (1) bilden soll,
wobei der einteilige Rohling und/oder das einteilige Fertigteil vorzugsweise aus Polymer, mehr bevorzugt aus Polyetheretherketon (PEEK) besteht.

## Claims

1. A surgical implant (1) for correcting the mutual positioning or orientation of the bones of a patient designed to be inserted between at least two bone bodies (2, 3) of said patient, said surgical implant (1) comprising:
- a main body (5) extending along a longitudinal axis (X-X') between a proximal end (6) and a distal end (7) of said main body (5),
- a plurality of fins (9), each fin (9) being provided with a junction edge (10) connecting said fin (9) to said main body (5), such that said fins (9) protrude from said main body (5) from the respective junction edge (10) thereof, which substantially extends in a junction plane (Pj) whose normal is formed by said longitudinal axis (X-X'), each fin (9) comprises a free edge (11) and extends between the junction edge (10) and the free edge (11),
the surgical implant (1) being **characterized in that** each fin (9) has a sufficient flexibility so that it can be deformed along a direction centripetal and/or parallel to the longitudinal axis (X-X') under the action of at least one of the bone bodies (2, 3) when said surgical implant (1) is inserted between said bone bodies (2, 3) and **in that** each fin (9) has at least:
- one portion of constant width (12) along which the free edge (11) and the junction edge (10) are separated by a first distance (d1) substantially constant along said portion of constant width (12) and,
- one flat portion (13), along which the free edge (11) and the junction edge (10) are separated by a second distance (d2) lower than the first distance (d1).

2. The surgical implant (1) according to the preceding claim, **characterized in that** it forms one single piece, made for example by molding, injection, or machining of a one piece blank.

3. The surgical implant (1) according to the preceding claim, **characterized in that** the main body (5) has a flexibility lower than the flexibility of the fins (9), so as to be able to deform under the action of at least one of the bone bodies (2, 3) when said surgical implant (1) is inserted between said bone bodies (2, 3) in the case where at least one of the fins (9) is completely deformed.

4. The surgical implant (1) according to any one of the preceding claims, **characterized in that** at least one of said fins (9) extends over at least the majority of, if not all, the perimeter of the main body (5) so as to surround the longitudinal axis (X-X').

5. The surgical implant (1) according to any one of the preceding claims, **characterized in that** the fins (9) are inclined relative to the longitudinal axis (X-X') so as to protrude from the main body (5) towards the proximal end (6), with an angle of inclination comprised between 45 and 90° relative to the longitudinal axis (X-X').

6. The surgical implant (1) according to any one of the preceding claims, **characterized in that** each fin (9) has at least two flat portions (13), along each of which the free edge (11) has a straight portion (13A) to form a straight flat portion (13), the flat portions (13) being regularly distributed around the longitudinal axis (X-X'), preferably symmetrically relative to each other.

7. The surgical implant (1) according to any one of claims 1, or 3 to 6, **characterized in that** it further comprises:
- a proximal radio-marker member (17) disposed within the main body (5) in the vicinity of the proximal end (6), and
- a distal radio-marker member (18) disposed within the main body (5) in the vicinity of the distal end (7),said proximal and distal radio-marker members being radio-marker wires, which are disposed within the main body (5) with an orientation distinct from each other.

8. The surgical implant (1) according to any one of the preceding claims, **characterized in that** it further comprises a through opening (14) formed in the main body (5) along the longitudinal axis (X-X').

9. The surgical implant (1) according to the preceding claim, **characterized in that** the through opening (14) includes an inner threading (16) extending from the proximal end (6).

10. The surgical implant (1) according to any one of claims 8 or 9, **characterized in that** the through opening (14) includes at least one interface notch (15) intended to cooperate with a setting-up tool (4) of the surgical implant (1), so that said setting-up tool (4) may drive the surgical implant (1) in rotation when it is inserted into the through opening (14) in cooperation with said notch (15).

11. The surgical implant (1) according to any one of the preceding claims, **characterized in that** it is an implant for treating a pathology of the flat foot in said patient.

12. A surgical kit including:
- a surgical implant (1) according to any one of claims 1 to 11,
- a setting-up tool (4) of the surgical implant (1).

13. The surgical kit according to claim 12, **characterized in that** said setting-up tool (4) comprises:
- a head (21) adapted to rotatably cooperate with the surgical implant (1) about the longitudinal axis (X-X') of said surgical implant, and
- a gripping handle (19) through which a user can grasp said setting-up tool (4), said handle including a visual indicator (20) of the orientation of the surgical implant (1) about its longitudinal axis (X-X') when said setting-up tool (4) is in cooperation with said surgical implant (1).

14. A method for manufacturing a surgical implant (1) according to any one of claims 1 to 11, **characterized in that** it comprises:
- either a step for molding a one piece finished part intended to form the surgical implant (1),
- or a step for machining a one piece blank in order to form a one piece finished part intended to form itself the surgical implant (1),
which one piece blank and/or one piece finished part is preferably made of polymer, still preferably made of polyetheretherketone (PEEK).
